# EUROPEAN PATENT SPECIFICATION

(11) **EP 0 461 144 B1**
(45) Date of publication and mention of the grant of the patent: **22.05.1996**
(21) Application number: 90903838.2
(22) Date of filing: 27.02.1990
(51) Int. Cl.: C12P 1/04, C02F 3/34, C12N 1/20

(54) **PROCESS AND MEANS FOR THE MICROBIOLOGICAL REMEDIATION OF POLLUTED SOIL AND MICROORGANISMS FOR USE IN SAID PROCESS**
VERFAHREN UND MITTEL ZUR MIKROBIOLOGISCHEN SANIERUNG VON VERUNREINIGTEM BODEN UND MIKROORGANISMEN ZUR VERWENDUNG IN DIESEM VERFAHREN
PROCEDE ET MOYEN POUR APPORTER UN REMEDE MICROBIOLOGIQUE A LA TERRE POLLUEE ET DES MICRO-ORGANISMES POUR UTILISATION DANS LEDIT PROCEDE

(30) Priority: 28.02.1989 FI 890949
(43) Date of publication of application: 18.12.1991
(73) Proprietor: Alko Group Ltd., SF-00101 Helsinki (FI)
(72) Inventor: VALO, Risto, Juhani, SF-02320 Espoo (FI); HÄGGBLOM, Max, Mikael, SF-00250 Helsinki (FI); SALKINOJA-SALONEN, Mirja, SF-00600 Helsinki (FI)
(74) Representative: Davies, Jonathan Mark
(86) International application number: FI9000055
(87) International publication number: WO9010079

(56) References cited:
- WO-A-90/01465
- DE-A- 3 617 875
- FI-A- 0 883 685
- APPLIED & ENVIRONMENTAL MICROBIOLOGY, vol. 54, no. 12, 1988; M.M. HAGGBLOM et al., pp. 3043-3052/
- APPLIED MICROBIOLOGY & BIOTECHNOLOGY, vol. 25, 1986; R. VALO et al./
- CHEMICAL ABSTRACTS, vol. 105, no. 5, 04 August 1986, Columbus, OH (US); J.H.A. APALAJAHTI et al., p. 411, no. 39171t/
- CHEMICAL ABSTRACTS, vol. 108, no. 5, 01 February 1988, Columbus, OH (US); J.H. A. APAJALAHTI et al., p. 332, no. 34580c/
- CHEMICAL ABSTRACTS, vol. 108, no. 25, 20 June 1988, Columbus, OH (US); J.H.A. APAJALAHTI et al., p. 302, no. 218784d/
- CHEMICAL ABSTRACTS, vol. 109, no. 16, 17 October 1988, Columbus, OH (US); M.M. HAGGBLOM et al., p. 337, no. 134439c/
- CHEMICAL ABSTRACTS, vol. 106, no. 19, 11 May 1987, Columbus, OH (US); J.H.A. APAJAHLAHTI et al., p. 368, no. 152724j/
- CHEMICAL ABSTRACTS, vol. 106, no. 19, 11 May 1987, Columbus, OH (US); R. VALO et al., p. 269, no. 151604h/

## Description

The invention relates to a process for the remediation of soil polluted by chlorinated phenolic compounds with the aid of microorganisms capable of degrading chlorinated phenolic compounds. The invention also relates to the new microorganisms to be used in said process as well as to said microorganisms immobilized in a solid support.

Chemically polluted soil and water is becoming an increasingly serious environmental problem in the industrialized countries. Chlorinated phenolic compounds and their derivatives, which are referred to in the following specification and claims by the term "chlorophenols" for short, are toxic hazardous contaminants which are hardly degradable in the environment. In Finland chlorophenol contaminated soil causes problems at the sites of saw mills where said chlorophenols have been used and where concentrations up to 5000 to 10 000 mg per kg dry soil have been located. Chlorophenols have also been found in surface water and in ground water in environmentally hazardous contents.

US Patent 4, 713, 340 (Crawford) describes the purification of chlorophenol contaminated surface and ground water by using bacteria of the genus Flavobacterium deposited under No. ATCC 39723 and capable of degrading chlorophenols. The same Patent also discloses that polluted soil may be purified by leaching the chlorophenols from the soil and removing said chlorophenols from the resulting polluted water.

The purification of polluted water with the aid of chlorophenol-degrading microorganisms has been disclosed in the copending international patent application PCT/FI89/00144 (based on the Finnish patent application 883685 filed August 8, 1988) which is incorporated herein by reference. Said application discloses a process for the microbiological purification of polluted water by a contaminant-degrading microorganism, in which process chlorophenols are enriched in a chlorophenol accumulating solid support and microorganisms which are immobilized in said support are made to degrade the accumulated chlorophenols. Polyurethane is the preferred support and biofilter which reversibly captures chlorophenols from the water. Said polyurethane may be specifically modified for the immobilization of microorganisms. As the chlorophenol-degrading microorganisms immobilized in the support the water purification process preferably uses chlorophenol-degrading bacteria of the genus Rhodococcus, which are capable of very effectively degrading the chlorophenols in the water.

Other known chlorophenol-degrading microorganisms are for instance some Arthrobacter and Pseudomonas bacteria, which have been disclosed by Portier et al., in Toxicity Assessment: An International Quarterly, Vol 1, p. 501...513, (1986).

The removal of chlorophenols from polluted soil is a much more difficult problem than their removal from water. Several chlorophenol-degrading microorganisms are prior known which are capable of degrading chlorophenols in favourable conditions in water or in a sterilized soil sample in a laboratory. It has, however, been found that it is very difficult to keep these known microorganisms alive in natural conditions in the soil and thus enable them to degrade the chlorophenols in said soil.

Although chlorophenols are toxic to humans, contaminated soil includes plenty of microorganisms which tolerate chlorophenols. Some of these do not affect chlorophenols at all, some transform chlorophenols e. g. by methylating them whereby the resulting chloroanisoles may be even more hazardous to humans than the actual chlorophenols. Only a part of the microorganisms which tolerate chlorophenols are ones which are capable of using chlorophenols as their carbon source.

Even though contaminated soil may include naturally occurring microorganisms which in laboratory conditions are capable of degrading chlorophenols, it has, however, been found that chlorophenols remain almost undegraded in soil. The natural conditions are generally such that they do not favour chlorophenol-degrading microorganisms.

Chlorophenol-degrading microorganisms which occur naturally in contaminated soil have been used for decontaminating polluted soil (Valo et al., Appl. Microbiol. Biotechnol. 1986, 25:68-75). In said experiment a naturally occurring microbe population was made to function by raising the temperature of the contaminated soil, adding nutrients to the soil and adjusting the pH close to neutral.

In a parallel experiment in the above mentioned paper it was noted that the degradation of chlorophenols in laboratory conditions accelerated when a pure culture of the chlorophenol-degrading microorganism Rhodococcus chlorophenolicus PCP-1 DSM 43826 was added to a sterilized contaminated soil sample. Said microorganism is described in detail by Apajalahti et al. in Int. J. Syst. Bacteriol., (1986) 36: 246 - 251. In the field scale experiment, however, it was found that the addition of chlorophenol-degrading bacteria in an amount of 1 x 10⁵ cells/g of contaminated soil did not have any significant effect.

As mentioned above several chlorophenol-degrading microorganisms are incapable of competing for the vital function's with other more quickly multiplying microorganisms. When such microorganisms are added to a foreign environment they are therefore generally incapable of effectively degrading chlorophenols. Moreover, the temperature of the soil is so low that it does not correspond to the temperature of bioactivity of the microorganisms (generally about 20 to 35 °C).

It has now been found that it is possible to isolate from soil or water certain chlorophenol-degrading microorganisms of the strains Rhodococcus and Mycobacterium which are stable in soil also in naturally occurring conditions. It has, however, been found that the chlorophenol-degrading activity of said microorganisms is so low that without external means they are incapable of decreasing the chlorophenol content of contaminated soil to any significant degree.

In connection with the present invention the conditions for the activity of said chlorophenol-degrading microorganisms were studied and hereby the means were discovered by which the said microorganisms can be made to work effectively so that the contaminated soil can be reclaimed.

The object of the present invention is to provide a process, by which chlorophenol contaminated soil can be efficiently reclaimed with the aid of chlorophenol-degrading microorganisms.

The object of the invention is also to provide a support material for use in said process which support comprises an immobilized microorganism.

The object of the invention is further to provide new microorganisms which are isolated from the environment and which preferably can be used in said process.

The more detailed objects of the invention will appear from the following specification as well as from the appended claims.

Thus, the object of the invention is a process for the microbiological remediation of contaminated soil from chlorinated phenolic compounds and their derivatives with the aid of chlorophenol-degrading microorganisms, in which process chlorophenol-degrading microorganisms belonging to the genus Rhodococcus and/or Mycobacterium are added to the contaminated soil and the facilities for the chlorophenol-degrading activity of the used microorganisms are improved by adjusting the growth conditions of said microorganisms, while avoiding the addition of an easily metabolized carbon source.

In order to improve the viability of said microorganisms in the conditions of the soil to be decontaminated the invention immobilizes them in a solid porous organic support, preferably in polyurethane which is especially modified for this purpose.

According to the invention it has also been found important to improve the facilities for the bioactivity of the microorganisms by adjusting the physical and/or chemical parameters of the soil, such as temperature, pH, moisture, redox potential, nutrients, aeration, etc. In this connection it has surprisingly been discovered that when adding nutrients one should avoid adding a carbon source which is easily utilizable by the microorganisms.

The present invention also relates to a modified polyurethane support which contains immobilized chlorophenol-degrading microorganisms, said support being characterized in that chlorophenol-degrading microorganisms of the genus Rhodococcus and/or Mycobacterium are immobilized into the polyurethane resin during the production of the polymer.

The invention further relates to microorganisms which are isolated from the environment and which degrade chlorinated phenols and their derivatives, and which microorganisms are:

Rhodococcus sp. CG-1, which has been deposited in accordance with the Budapest Treaty on January 10, 1989 in the depository DSM (Deutsche Sammlung von Mikroorganismen und Zellkulturen GmbH) and obtained the number DSM 5146, and
Mycobacterium sp. CG-2, which has been deposited in accordance with the Budapest Treaty on January 10, 1989 in the depository DSM (Deutsche Sammlung von Mikroorganismen und Zellkulturen GmbH) and obtained the number DSM 5129.

The invention is further illustrated by the following specification and the appended drawings, wherein

Fig 1 shows the effect of the addition of a carbon source on the evolution of carbon dioxide indicating the degradation of pentachlorophenol, PCP, responsive to the chlorophenol-degrading activity of a microorganism immobilized in accordance with the present invention.

Fig 2 shows the degradation of ¹⁴C labeled pentachlorophenol, PCP, responsive to the bioactivity of a microorganism according to the invention.

According to the process of the present invention chlorophenols are removed biologically from contaminated soil by adding a sufficient amount, preferably at least 1 x 10⁶ cells/g dry soil of chlorophenol-degrading microorganisms of the genus Rhodococcus and/or Mycobacterium and by at the same time adjusting the conditions of bioactivity for said microorganisms so that an effective microbial activity is possible.

In the invention it has been found especially preferred to add the microorganisms immobilized in a solid porous organic support. The support forms a favourable living environment for the microorganisms and it protects the microorganisms from the attack of predatory beings. It is further preferred that the porous support is capable of accumulating chlorophenols from the liquid phase of the surrounding soil and thus lowering the chlorophenol concentration in the immediate living environment of the microorganisms. This is especially important when it is a question of the microorganisms of the genus Rhodococcus and Mycobacterium used according to the invention since the tolerance of said microorganisms for chlorophenols is relatively low and their activity is thus improved when the support lowers the chlorophenol content of the water phase of the soil. When the chlorophenol content decreases due to the microbial activity, chlorophenols migrate back from the support into the soil for degradation by the microorganisms.

Porous materials, especially porous organic materials such as wood chips and wood bark and polyurethane are suitable as support materials since they have been found to function well as attachment substrates for the microorganisms and they accumulate chlorophenols.

According to the invention polyurethane resins which are capable of effectively acting as immobilization substrates for the degrading microorganisms are especially well suited as supports. The polyurethanes to be used have a large specific area and a large porosity. The utilized polyurethane resin may be of a special polyurethane kind which is modified for the immobilization of bacteria and which is capable of accumulating chlorophenols due to its large surface area and the active carbon that it contains as well as due to its surface charge.

Chips of wood and wood bark have been found to reversibly accumulate chlorophenols from chlorophenol containing solutions (see Apajalahti et al. Microbiol. Ecol. (1984) 10: 359... 367) and to function very well also as immobilization substrates for microorganisms.

Such supports comprising immobilized chlorophenol-degrading microorganisms are disclosed in the previously mentioned international patent application PCT/FI89/00144 and they can be used also in the process of the present invention.

In the scope of the present invention it has further been found that the immobilization of the microorganisms into the polyurethane support may be performed during the production of the support material. The immobilization is then performed during the polymerization of the polyurethane for instance by adding living cells into a mixture of polyethylene glycol and an isocyanate compound. During the polymerization whole cells form a part of the actual polymer. The immobilization of microorganisms by polymerization and polycondensation are known immobilization techniques as such, as is made evident by the book by Klein, J., Wagner. F, Applied Biochemistry and Bioengineering, Vol. 4, in the part called Methods for the Immobilization of Microbial Cells, p. 25 to 31.

When encapsulated within the polyurethane the microorganisms are especially well protected for the purpose of being used according to the present invention. Moreover, the actual polyurethane may be modified to suit the needs of the microorganisms and the chemical to be treated.

The bioactivity of the contaminant-degrading bacteria and especially their degrading rate depends on the temperature. In field conditions the problem with the reclamation of contaminated soil often is that the bacterial activity at a temperature below 15 °C is low and stops completely below 8 °C. In an embodiment of the invention the required heating is provided for instance by allowing for a heat producing composting to take place and by ascertaining that the size and form of the pit are heat energetically favourable and/or by heating the pit by external heating, such as electric resistor elements or the like. The amount of composting material to be added depends on the natural content of organic material in the soil to be treated as well as on the need to raise the temperature. A suitable amount of digestible organic material is generally about 0.1 to 1 %.

The rotting organic material, such as wood bark, park waste etc. functions as an oxygen consuming agent besides providing heat and porosity. Thus the redox potential of the bioactivity is controlled. In accordance with the invention it has been found that the chlorophenol-degrading activity of the said microorganisms is favoured by a low oxygen content in the soil. Thus for instance 1 % air does not contain a harmful amount of oxygen, while on the other hand there is an excessive amount of air for the microorganisms in case the soil contains more than 50 % air. The soil to be decontaminated is therefore preferably isolated from the environment by a plastic film. This also prevents rain water from penetrating into the compost and chlorophenol containing water from leaking to the lower soil layers. The soil to be decontaminated is preferably mixed at intervals in order to bring the microorganisms into contact with fresh portions of the soil.

For the bioactivity of the microorganisms the pH is adjusted to over 6, preferably close to 7 to 8. The contaminated soil may be buffered for instance by the addition of ash which at the same time comprises a mineral nutrient. In accordance with the invention it has been found that nitrogen and phosporus should generally be added to the soil.

In the addition of nutrients according to the invention it has surprisingly been found that the addition of an easily metabolized carbon source favours the o-methylation of the chlorophenols and weakens the mineralization of the chlorophenols to CO₂ and inorganic chloride. Thus it is extremely important that no easily metabolized carbon source is added to the soil to be decontaminated.

In order to maintain a sufficient gas circulation in the soil said soil must not be too moist. On the other hand the microorganism activity is not either at its maximum in too dry soil. In the soil reclamation according to the invention it has been found preferable to adjust the moisture of the soil to a value which is close to 60 % of the water holding capacity, WHC, of the soil.

Microorganisms which are useful in the process of the invention are chlorophenol-degrading microorganisms of the genus Rhodococcus and Mycobacterium which comprise for instance the following identified strains:
Rhodococcus chlorophenolicus PCP-1 (DSM 43826), which has been described by Apajalahti et al. in Int. J. Syst. Bacteriol., (1986) 36: 246... 251, and which has been re-deposited according to the Budapest Treaty on January 10, 1989 in the depository DSM (Deutsche Sammlung von Mikroorganismen und Zellkulturen GmbH) and obtained the number DSM 5128;
Rhodococcus sp. CP-2, which is disclosed in the international patent application PCT/FI89/00144 as well as in Häggblom et al. Appl. Envir. Microbiol (1988) 54: 3043... 3052, and which has been deposited according to the Budapest Treaty on May 13, 1988 in the depository DSM and obtained the number DSM 4598;
Rhodococcus sp. CG-1, which has been deposited according to the Budapest Treaty on January 10, 1989 in the depository DSM and obtained the number DSM 5146; and
Mycobacterium sp. CG-2, which has been deposited according to the Budapest Treaty on January 10, 1989 in the depository DSM and obtained the number DSM 5129.

Said microorganisms were isolated from chlorophenol contaminated soil and water and it is obvious to a person skilled in the art that other corresponding microorganisms may be isolated in a corresponding way and that such may be used in the process of the invention. It is also clear that chlorophenol-degrading cultures derived from said microorganisms are suitable for being utilized in the process of the invention. The characteristics of the strains Rhodococcus chlorophenolicus PCP-1 and Rhodococcus sp. CP-2 have been described in the above mentioned literature references, which are included herein by reference.

The strains Rhodococcus CG-1 and Mycobacterium CG-2 which are disclosed as new microorganisms according to the invention have also been described by Häggblom et al. in Appl. Envir. Microbiol (1988) 54: 3043... 3052. The reference does not, however, include information as to the deposition of the microorganisms and their identification is therefore not so clear from the said reference that a person skilled in the art could be sure to isolate them and thus obtain them for use. The said microorganisms are therefore to be regarded as being new microorganisms which now by the performed deposition have been disclosed in such a way that they can be regarded as being identified and reproducible. As regards the general characteristics and the chlorophenol-degrading characteristics of the new microorganisms reference is made to the above mentioned paper.

The probable degradation path of pentachlorophenol to carbon dioxide and inorganic chloride by the action of bacteria of the genus Rhodococcus and Mycobacterium is shown in the following reaction scheme: 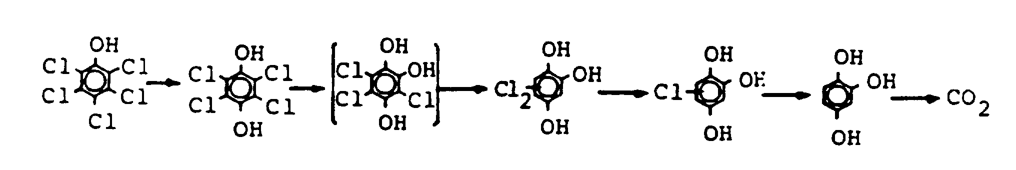 The chlorophenol-degrading capacity of the bacteria Rhodococcus CP-2 and Rhodococcus chlorophenolicus PCP-1 is genetically stable while the degrading capacity of the bacteria Rhodococcus CG-1 and Mycobacterium CG-2 is instable and vanishes in case the substrate lacks chlorinated phenols. The capacity for degrading chlorinated phenols may be induced into the bacteria either prior to the immobilization or after the immobilization. In connection with the induction the bacteria are fed with the chemical to be degraded in connection with a nutrient medium so that the bacteria will produce the enzymes which degrade the specific contaminant.

The following Examples illustrate the invention without, however, limiting it in any way.

### Example 1

### The isolation of chlorophenol-degrading microorganisms

Two 500 ml columns with softwood bark chips as a solid support were inoculated with 10 to 15 g aliquots of a contaminated slurry (sample A) and soil (sample B) each, and 200 ml of a mineral salts medium was percolated through the columns. Tetrachloroguaijacol (TeCG) was weekly added up to a concentration of 10 to 50 µM. After 3 months, the percolating fluids were found to contain a mixed culture that repeatedly removed added TeCG (10 µM). The A and B cultures from the percolators were enriched by several dilutions and feeding with 10 to 20 µM TeCG added at 2 to 3 days intervals. Samples of A and B were streaked onto DSM-65 (glucose 4.0 g, yeast extract 4.0 g, malt extract 4.0 g, H₂O 1000 ml, pH 7.2) agar with 10 µM TeCG. 130 isolates from each mixed culture were tested for TeCG degradation, by monitoring the removal of TeCG from the liquid. From the culture A was isolated one TeCG degrading colony and it was designated CG-1 and correspondingly a colony isolated from B was designated CG-2.

A third chlorophenol degrading culture (C) was correspondingly obtained from a chlorophenol contaminated soil. The culture in minerals salts medium was enriched for a period of 8 months by repeated dilutions and feeding with PCP (5 mg/l). The culture was plated on yeast extract agar containing PCP (20 mg/l), and one colony that degraded PCP was chosen and designated CP-2.

By corresponding methods also other chlorophenol-degrading Rhodococcus and Mycobacterium bacteria can be isolated and such bacteria may be used in the method and product of the present invention.

The pentachlorophenol-degrading capacity of the isolated microbe strains was measured by using ¹⁴C labeled PCP and by monitoring the evolved ¹⁴CO₂. The degradation of other chlorophenols was also measured.

The strain CG-1 (Rhodococcus sp. CG-1) degraded PCP, 2346-TeCP, 2356-TeCP, 235-TCP, 236-TCP, 246-TCP, 25-DCP, 34-DCP, TeCG, 346-TCG, 346/356 TCG and TCS in 48 hours.

The strain CG-2 (Mycobacterium sp. CG-2) completely degraded PCP, 2345-TeCP, 2346-TeCP, 2356-TeCP, 234-TCP, 235-TCP, 236-TCP, 246-TCP, 24-DCP, 26-DCP, TeCG, 345-TCG, 346-TCG, 346/356-TCG, 456-TCG, 34-DCG, 35-DCG, 36-DCG, TCS and 35-DCS.

CP-2 (Rhodococcus sp. CP-2) also degraded several other chlorinated phenols, guajacols and syringols in 10 µM solutions. In said tests PCP, 2345-TeCP, 2346-TeCP, 2356-TeCP, 234-TCP, 235-TCP, 236-TCP, 245-TCP, 25-DCP, TeCG, 345-TCG, 346-TCG, 356-TCG, 456-TCG, 34-DCG, 35-DCG, 36-DCG, TCS and 35-DCS were degraded in 48 hours.

The above used abbreviations refer to the chlorophenols in the following way: pentachlorophenol (PCP), tetrachlorophenol (TeCP), trichlorophenol (TCP), dichlorophenol (DCP), tetrachloroguajacol (i.e. tetrachloro-2-methoxyphenol) (TeCG), trichloroguajacol (TCG), and trichlorosyringol (i.e. trichloro-2, 6-dimethoxyphenol) (TCS).

### Example 2

### Immobilization of the microorganism in the support

Pure cultures of the chlorophenol-degrading microorganisms Rhodococcus chlorophenolicus PCP-1 (DSM 5128) and Rhodococcus CP-2 (DSM 4598), were immobilized in a polyurethane resin mass of the polyurethane REA 90/16 produced by Bayer Ag, West Germany. Said polyurethane is modified for the immobilization of bacteria (polyurethane particle size below 10 mm; density 1.14 to 1.05 kg/l at 20 °C; weight in suspension 115 kg dry matter per m³ of suspension volume; sedimentation rate 94 ± 6 m/h).

A mixed culture of the bacteria was incubated in a fermentor using 1% glucose, 1% soritol as a carbon source and in the presence of a support. As they grew the bacteria excreted slime and attached perceptively onto the surface of the support material. When sufficient biomass had formed the incubation was ended.

The polyurethane mass containing the immobilized Rhodococcus culture was stored for 20 days at +4 °C. In a test performed after said storage the microbes were found to be still capable of degrading chlorophenols.

Chlorophenol-degrading Rhodococcus bacteria have been found to degrade chlorophenols even after having been stored at +4 °C for one year.

### Example 3

### The degradation of chlorophenols in a soil sample

Two soil samples were used in the test, one of an organic soil and the other of a sandy soil. The unsterilized soil samples were deliberately contaminated with PCP (30 g/l in 0.15 M NaOH) to a concentration of 665 mg/kg dry sandy soil and 1860 mg/kg dry organic soil.

As the chlorophenol-degrading bacterium (degrader) the strain Rhodococcus chlorophenolicus PCP-1 (DSM 5128) was used and as the o-methylating bacterium (methylater) the strain Rhodococcus rhodochrous (DSM 43241) was used. The strains were grown in DSM-65 medium for a week in the presence of sterile polyurethane, PU, (REA 90/16, Bayer, Leverkusen, West Germany). The Rhodococcus chlorophenolicus cultures were induced with PCP daily up to a concentration of 50 µM.

In the inoculation of the microorganisms 0. 5 g (dry weight) of the resulting polyurethane containing the immobilized cells was used. Added to the soils this gave a concentration of 1 x 10⁸ cells/g dry weight for the sandy soil and 2. 5 x 10⁸ cells/g dry weight for the organic soil. Sterile PUwas used as a control to indicate whether the addition of organic matter as such affects the degradation of PCP.

Glucose/yeast extract was used as an easily metabolized carbon source in the form of a solution of 25 % by weight of glucose and 25 % by weight of yeast extract in water. The test included a total of the following 10 samples:

| | | Glucose/yeast extract |
|---|---|---|
| 1) | No inoculation | - |
| 2) | No inoculation | + |
| 3) | Sterile PU | - |
| 4) | Sterile PU | + |
| 5) | Inoc. with methylaters | - |
| 6) | Inoc. with methylaters | + |
| 7) | Inoc. with degraders | - |
| 8) | Inoc. with degraders | + |
| 9) | Inoc. with meth. and degr. | - |
| 10) | Inoc. with meth. and degr. | + |

All inoculations were made using 1 % by weight of dry polyurethane calculated on the basis of moist soil. Glucose/yeast extract was added to a concentration 0. 5 % each relative to the moist soil.

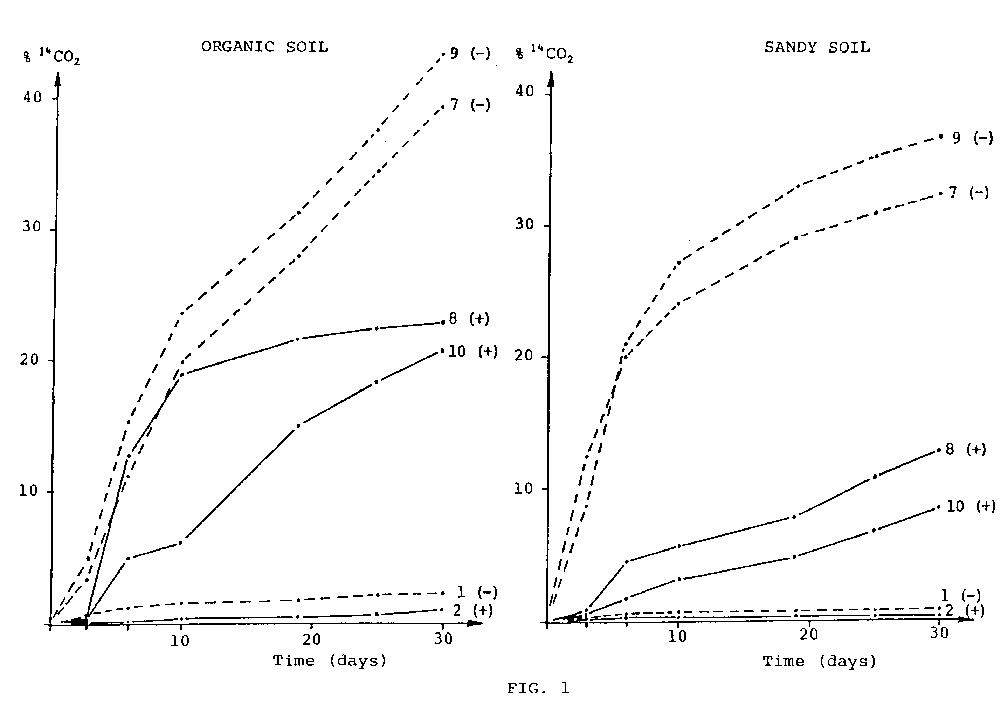

All tests were duplicated with added ¹⁴C-labeled PCP. From said tests the degradation of PCP was monitored on the basis of the percentage of evolved ¹⁴CO₂. The results are shown in Fig. 1, wherein the tests performed with sterile PU and the methylaters have been left out for clarity, as the results of said tests essentially followed the same pattern as the corresponding uninoculated soils. In the Fig. the graphs are numbered according to the numbering of the above Table whereby the (+) and (-) marks designate the presence and the absence of a carbon source, respectively. The results clearly indicate that the used microorganism is capable of degrading PCP in unsterilized soil and that the addition of an easily metabolized carbon source decreases the mineralization of PCP.

### Example 4

A test corresponding to Example 3 was performed on soil samples (samples A and B) taken from two different sites of chlorophenol contaminated soil. A cell suspension of the degrading microorganism Rhodococcus CP-2 was added to said samples in a concentration of 1.1 x 10⁷ cells/g dry soil. In order to indicate degradation, ¹⁴C-labeled PCP was added to the soil samples and the degradation thereof was monitored by measuring the amount of ¹⁴CO₂ produced. To some of the samples glucose/yeast extract was added in a concentration of 1 % as an easily metabolized carbon source.

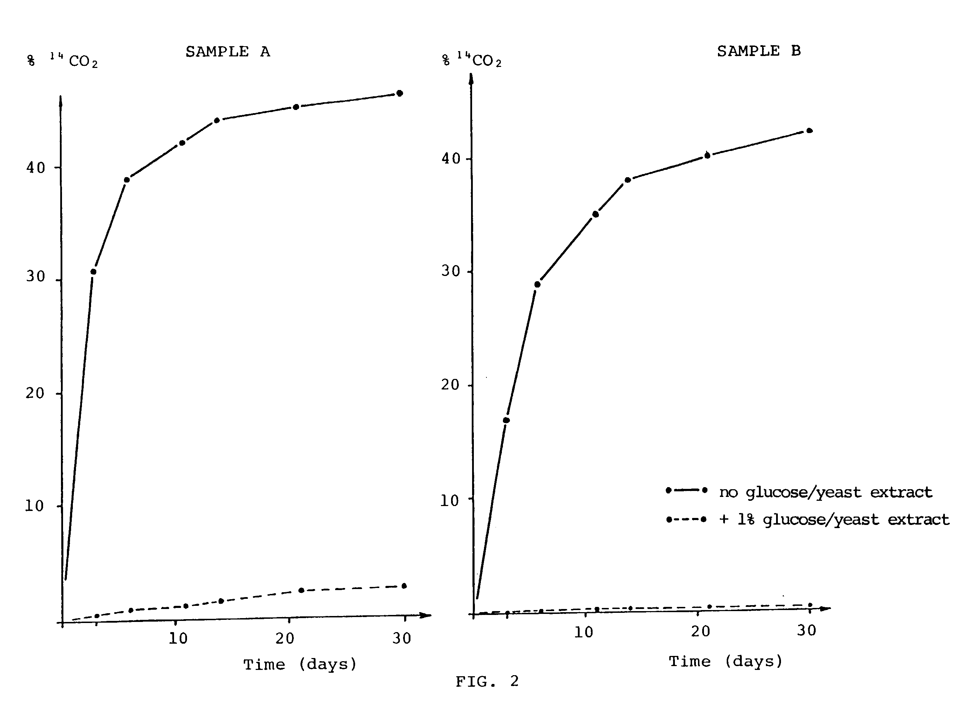

The results are shown in Fig. 2 which clearly indicates the negative effect of an easily metabolized carbon source on the mineralization of PCP.

### Example 5

### Field experiment

Chlorophenol contaminated soil is moved to a plastic lined windrow and the following materials are mixed therein
- wood bark and park refuse in order to raise the content of digestible organic matter to about 0. 5 %;
- ash for buffering the pH to a value of about 7;
- fertilizer in an amount of about 1 kg/m³ dissolved in water, said fertilizer containing about 16 % N, about 7 % P and about 3.3 % K;
- water at need in order to adjust the moisture level to about 60 % of the water holding capacity.
Microorganisms Rhodococcus chlorophenolicus PCP-1 and/or Rhodococcus CG-1 immobilized in polyurethane are also mixed into said soil in a concentration of about 10⁸ cells/g dry soil.

After mixing the soil is covered with a plastic film. The temperature of the windrow is monitored during the composting and in case the heath obtained from the digestion is not sufficient to raise the temperature of the windrow to about 20 to 25°C an additional heating device mounted in the bottom of the windrow is used for heating. The soil is mixed and its chlorophenol content is measured about once a month. When the chlorophenol content of the soil has decreased to a predetermined acceptable level the windrow is broken up and the reclaimed soil is moved away.

## Claims

1. A process for the microbiological remediation of polluted soil from chlorinated phenolic compounds and their derivatives with the aid of chlorophenol-degrading microorganisms, **characterized** in that chlorophenol-degrading microorganisms of the genus Rhodococcus and/or Mycobacterium are added immobilized in a solid porous support to the contaminated soil in cell counts which are sufficient to provide a chlorophenol-degrading bioactivity and that the facilities for the bioactivity of the used microorganisms are improved by adjusting the growth conditions for said microorganisms, while avoiding the addition of an easily metabolized carbon source.

2. A process according to claim 1, **characterized** in that the microorganisms are immobilized in a polyurethane resin, in wood bark or in wood chips.

3. A process according to claim 2, **characterized** in that the microorganism are immobilized in a polyurethane resin by incubating said microorganisms in a polyurethane containing medium or by producing said polyurethane from a microorganism containing medium.

4. A process according to one of the preceding claims 1 to 3, **characterized** in that the facilities for the bioactivity of said microorganisms are improved by adjusting the physical and/or chemical parameters of the soil, said parameters being chosen from e.g. temperature, pH, moisture, redox potential, nutrients, aeration.

5. A process according to claim 4, **characterized** in that the temperature and the redox potential are adjusted by adding to the soil organic matter, such as microbiologically digestible wood bark, park refuse or the like and/or that the soil to be treated is enclosed in a impermeable plastic film to decrease the aeration.

6. A process according to any one of the preceding claims, **characterized** in that the microorganisms are chosen from the group consisting of the chlorophenol-degrading microorganisms Rhodococcus chlorophenolicus PCP-1 (DSM 5128), Rhodococcus sp. CP-2 (DSM 4598), Rhodococcus sp. CG-1 (DSM 5146) and Mycobacterium sp. CG-2 (DSM 5129).

7. A polyurethane support modified for the immobilization of bacteria which support contains immobilized chlorophenol-degrading microorganisms, **characterized** in that chlorophenol-degrading microorganisms of the genus Rhodococcus and/or Mycobacterium are immobilized in said polyurethane resin during the production of the polymer.

8. A polyurethane support according to claim 7, **characterized** in that the microorganisms are chosen from the group consisting of the chlorophenol-degrading microorganisms Rhodococcus chlorophenolicus PCP-1 (DSM 5128), Rhodococcus sp. CP-2 (DSM 4598), Rhodococcus sp. CG-1 (DSM 5146) and Mycobacterium sp. CG-2 (DSM 5129).

9. A pure culture of a microorganism which is capable of degrading chlorinated phenolic compounds and their derivatives, **characterized** in that said microorganism is Rhodococcus sp. CG-1 (DSM 5146) or a culture derived therefrom.

10. A pure culture of a microorganism which is capable of degrading chlorinated phenolic compounds and their derivatives, **characterized** in that said microorganism is Mycobacterium sp. CG-2 (DSM 5129) or a culture derived therefrom.

## Patentansprüche

1. Verfahren zur mikrobiologischen Dekontamination von verunreinigtem Boden von chlorierten Phenolverbindungen und ihren Derivaten unter Zuhilfenahme von Chlorphenol abbauenden Mikroorganismen, dadurch gekennzeichnet, daß in einem festen porösen Träger immobilisierte, Chlorphenol abbauende Mikroorganismen der Gattung Rhodococcus und/oder Mycobacterium in einer Zellzahl dem kontaminierten Boden zugesetzt werden, die zur Bereitstellung von Chlorphenol abbauender Bioaktivität ausreicht, und daß die Fähigkeiten zur Bioaktivität der verwendeten Mikroorganismen durch das Einstellen der Wachstumsbedingungen für die Mikroorganismen verbessert werden, während die Zugabe einer leicht metabolisierbaren Kohlenstoffquelle vermieden wird.

2. Verfahren nach Anspruch 1, dadurch gekennzeichnet, daß die Mikroorganismen in einem Polyurethanharz, in Holzrinde oder in Holzspänen immobilisiert werden.

3. Verfahren nach Anspruch 2, dadurch gekennzeichnet, daß die Mikroorganismen in einem Polyurethanharz immobilisiert werden durch die Inkubation der Mikroorganismen in einem Polyurethan enthaltenden Medium oder durch die Produktion des Polyurethans von einem Mikroorganismen enthaltenden Medium.

4. Verfahren nach einem der vorangehenden Ansprüche 1 bis 3, dadurch gekennzeichnet, daß die Fähigkeiten zur Bioaktivität der Mikroorganismen durch das Einstellen der physikalischen und/oder chemischen Parameter des Bodens verbessert werden, wobei die Parameter ausgewählt sind aus Temperatur, pH-Wert, Feuchtigkeit, Redoxpotential, Nährstoffen und Belüftung.

5. Verfahren nach Anspruch 4, dadurch gekennzeichnet, daß die Temperatur und das Redoxpotential durch die Zufuhr von organischen Stoffen zum Boden eingestellt werden, wie mikrobiologisch abbaubare Holzrinde, Parkabfall oder ähnlichem, und/oder daß der zu behandelnde Boden zur Verminderung der Luftzufuhr in einer undurchlässigen Plastikfolie eingeschlossen ist.

6. Verfahren nach einem der vorangehenden Ansprüche, dadurch gekennzeichnet, daß die Mikroorganismen ausgewählt sind aus den Chlorphenol abbauenden Mikroorganismen Rhodococcus chlorophenolicus PCP-1 (DSM 5128), Rhodococcus sp. CP-2 (DSM 4598), Rhodococcus sp. CG-1 (DSM 5146) und Mycobacterium sp. CG-2 (DSM 5129).

7. Ein für die Immobilisierung von Bakterien modifizierter Polyurethanträger, der immobilisierte Chlorphenol abbauende Mikroorganismen enthält, dadurch gekennzeichnet, daß Chlorphenol abbauende Mikroorganismen der Gattung Rhodococcus und/oder Mycobacterium in dem Polyurethanharz während der Produktion des Polymers immobilisiert werden.

8. Polyurethanträger nach Anspruch 7, dadurch gekennzeichnet, daß die Mikroorganismen ausgewählt werden aus den Chlorphenol abbauenden Mikroorganismen Rhodococcus chlorophenolicus PCP-1 (DSM 5128), Rhodococcus sp. CP-2 (DSM 4598), Rhodococcus sp. CG-1 (DSM 5146) und Mycobacterium sp. CG-2 (DSM 5129).

9. Mikroorganismus-Reinkultur, die chlorierte Phenolverbindungen und ihre Derivate abbauen kann, dadurch gekennzeichnet, daß der Mikroorganismus Rhodococcus sp. CG-1 (DSM 5146) oder eine davon abstammende Kultur ist.

10. Mikroorganismus-Reinkultur, die chlorierte Phenolverbindungen und ihre Derivate abbauen kann, dadurch gekennzeichnet, daß der Mikroorganismus Mycobacterium sp. CG-2 (DSM 5129) oder eine davon abstammende Kultur ist.

## Revendications

1. Procédé pour le traitement microbiologique de la terre polluée par des composés phénoliques chlorés et leurs dérivés au moyen de micro-organismes dégradant les chlorophénols, caractérisé en ce que des micro-organismes dégradant les chlorophénols appartenant aux genres Rhodococcus et/ou Mycobacterium sont ajoutés à l'état immobilisé sur un support poreux solide à la terre contaminée en des numérations de cellules qui sont suffisantes pour engendrer une activité biologique de dégradation des chlorophénols, et en ce que les dispositifs permettant l'activité biologique des micro-organismes utilisés sont améliorés en ajustant les conditions de croissance desdits micro-organismes, tout en évitant l'addition une source de carbone métabolisée aisément.

2. Procédé suivant la revendication 1, caractérisé en ce que les micro-organismes sont immobilisés dans une résine de polyuréthanne, dans de l'écorce d'arbre ou dans des copeaux de bois.

3. Procédé suivant la revendication 2, caractérisé en ce que les micro-organismes sont immobilisés dans une résine de polyuréthanne en mettant en incubation lesdits micro-organismes dans un milieu contenant du polyuréthanne ou bien en produisant ledit polyuréthanne à partir d'un milieu contenant les micro-organismes.

4. Procédé suivant une des revendications 1 à 3 précédentes, caractérisé en ce que les dispositifs permettant l'activité biologique des micro-organismes sont améliorés en ajustant les paramètres physiques et/ou chimiques de la terre, lesdits paramètres étant choisis, par exemple, entre la température, le pH, l'humidité, le potentiel d'oxydoréduction, les substances nutritives et l'aération.

5. Procédé suivant la revendication 4, caractérisé en ce que la température et le potentiel d'oxydoréduction sont ajustés en ajoutant à la terre une matière organique, telle que de l'écorce d'arbre, des déchets d'entretien de jardins publics, etc., aptes à la digestion microbiologique, et/ou en ce que la terre à traiter est enfermée dans un film de matière plastique imperméable pour diminuer l'aération.

6. Procédé suivant l'une quelconque des revendications précédentes, caractérisé en ce que les micro-organismes sont choisis dans le groupe consistant en les micro-organismes dégradant les chlorophénols, Rhodococcus chlorophenolicus PCP-1 (DSM 5128), Rhodococcus sp. CP-2 (DSM 4598), Rhodococcus sp. GS-1 (DSM 5146) et Mycobacterium sp. CG-2 (DSM 5129).

7. Support en polyuréthanne modifié pour l'immobilisation de bactéries, support qui contient des micro-organismes immobilisés dégradant les chlorophénols, caractérisé en ce que des micro-organismes dégradant les chlorophénols appartenant aux genres Rhodococcus et/ou Mycobacterium sont immobilisés dans ladite résine de polyuréthanne au cours de la production du polymère.

8. Support en polyuréthanne suivant la revendication 7, caractérisé en ce que les micro-organismes sont choisis dans le groupe consistant en les micro-organismes dégradant les chlorophénols Rhodococcus chlorophenolicus PCP-1 (DSM 5128), Rhodococcus sp. CP-2 (DSM 4598), Rhodococcus sp. CG-1 (DSM 5146) et Mycobacterium sp. CG-2 (DSM 5129).

9. Culture pure d'un micro-organisme qui est capable de dégrader des composés phénoliques chlorés et leurs dérivés, caractérisée en ce que ledit micro-organisme consiste en Rhodococcus sp. CG-1 (DSM 5146), ou une culture qui en est dérivée.

10. Culture pure d'un micro-organisme qui est capable de dégrader des composés phénoliques chlorés et leurs dérivés, caractérisée en ce que ledit micro-organisme consiste en Mycobacterium sp. CG-2 (DSM 5129) ou une culture qui en est dérivée.
